# EUROPEAN PATENT APPLICATION

(11) **EP 1 913 887 A1**
(43) Date of publication of application: **23.04.2008**
(21) Application number: 06386035.7
(22) Date of filing: 20.10.2006
(51) Int. Cl.: A61B 17/72

(54) **Intramedullary leg lengthening nail using shape memory alloys**

(71) Applicant: Kostopoulos, Vassilis, 26500 Rio Patras (GR); Panagiotopoulos, Elias, 26442 Patras (GR); Tsantzalis, Stavros, 12462 Haidari Attikis (GR); Fortis, Athanasios, 22100 Tripoli Arkadias (GR)
(72) Inventor: Kostopoulos, Vassilis, 26500 Rio Patras (GR); Panagiotopoulos, Elias, 26442 Patras (GR); Tsantzalis, Stavros, 12462 Haidari Attikis (GR); Fortis, Athanasios, 22100 Tripoli Arkadias (GR)

(57) **Abstract**

The invention is reported in the design; manufacture and the application of intramedullary leg lengthening nail with variable length, manufactured from stainless steel or titanium alloy with the capability of continuous change of its length within predetermined limits and rhythm of elongation. The mechanism of movement /elongation is based on the use of shape memory alloy (7) which by its rotation, turns a mechanism of one way clutcher (8), which turns a screw (3) that moves the two parts (1, 2) in which are incorporated the two mechanisms. A spring (4) between the two parts (1, 2) helps to increase the gap without application of big torsional torque from the shape memory alloy. The activation of the shape memory alloys based on an induction heating system which will be applied on skin of the leg. The intramedullary nail will be used for therapeutic reasons i.e. to increase, after osteotomy, the length of the leg/member in which it has been implanted. The intramedullary nail will be very helpful to establish the loss of bone after car accident, or because of pathological conditions.

## Description

### Background of the Invention - existing Knowledge

The technique to increase the length of the long bones is the subject of research for the orthopedic surgeons for many years. The is used for the treatment of a limb shortening due to malformation or to a deficit for other reasons e.g. the fracture of a long bone after a car accident, osteomyelitis, or malignancy.

The procedure to increase the length of a bone is difficult and may become quite hazardous for the soft tissues surrounding the area. The two parts of the bone are stabilized to eliminate the possibility of relative torsion and bending. Then they aligned axially the one with respect to the other and moved with a constant rate of elongation 1mm per day, which is usually given in 4 steps of 0,25mm every 6 hours.

The mechanism, which ensures the above mentioned rate, must also be able to bear the compressive loads applied at the osteotomy site.

At the end of the '50s the most famous technique to increase the length of the bones, defined as distraction osteogenesis, was invented G.A Ilizarov (a Russian orthopedist). This technique consists of gradual displacement of the two parts of an osteotomized bone, through insertion of stainless steel wires, across the axis of bone and the fixation of these under tension to an external stainless ring. These rings the number of which varies according to the complexity of the fractured area from 4 to 6, are stabilized between them with stainless screws that in turn are secured with nut-screws that firstly ensure the sturdiness and stiffness of manufacture. Via the nut-screws the relative axial increase of the distance of the rings is allowed and by that increased the length of the bone way is controlled with the rates reported above. This method despite the disadvantages due to its initial design is still the most popular technique to increase the length of long bones and it is known in the literature as the Ilizarov method.

These disadvantages include:
- Infections occurring during the treatment from the wires crossing the soft tissues and the bone (pin track infections).
- Blood loss at the first days of application of the system
- Fixation loss due to problems of the mechanism by using transfixion wires as structural elements
- Fatigue of the wires during the treatment
- Failure to control with precision the alignment of the lengthened bone. That is wed to the fact that the motion of the two parts of the bone is ealized by moving the rings via the ties that are used. The whole process is repeated manually in three different places of the apparatus. Due to either patient loss of compliance or different on each side resistance to longation, the gap produced between the two parts of the bone may be asymmetrical and this can lead to non-union.
- Psychological distress of the patient because of the continuous pain and the view of the bulge apparatus, requiring permanent careful handling.

All the above disadvantages led to multiple redesigns of the apparatus.

However despite of the improvements that took place in the second generation of the devices for distraction osteogenesis, the disadvantages that the initial conception is related to were not eliminated.

All the above led the scientists to introduce new concepts (mechanisms) of distraction osteogenesis that would not be placed externally but inside the bone with explicit report to the use of experience of internal osteosynthesis by using nails.

The nails offer many advantages as - they avoid the complexity of the external apparatus and the wires which irritate the skin, producing thus pain and possibly infection. Maintenance of the alignment becomes easy and the elimination of infections as well as callus malformations are essential advantages. Furthermore the use of the intramedullary nail requires less patient compliance and the distress of the patient is insignificant. The basic idea of such mechanism includes a nail placed inside the bone, after an osteotomy, comprised of two parts, with the ability to increase its length at a constant rate.

In this case for the displacement of the two parts of the bone, the compressive forces that are applied by the soft tissues should be overcomed. The first effort introduced a mechanism where the increase of length of the intramedullary nail produced from the torsion of one of the parts as for the other via the torsion of the patient's leg based on his own forces. This effort even if overcame some of the disadvantages it actually failed because the activation of the mechanism via torsion of the leg it was quite laborious. Hence the patient at the first appearance of the pain refuses to turn the mechanism anymore, failing that way to activate it. In other cases the patients activated the mechanism more than once and that resulted to the creation of problematic quality of callous in the gap. Also, we may add the fact that the activation of the mechanism with relative torsion of the two parts, led to the creation of torsion strain at the osteotomy site.

The next generation of the mechanism of intramedullary distraction osteosynthesis was constituted by a tie which one side was incorporated in a mechanism of one way movement and the other was fixed to the interior of the bone. Simultaneously outside this tie another one existed which was fixed to the top and the bottom of the leg. Based on these two ties the displacement of the mechanism is achieved. However this did not function successfully because the fixations forced locally the bone with big forces and also because the top and the bottom side of the bone did not allow the free movement of the leg, as the screws were incorporated in them. The movement applied via suitable electrically driven mechanism that was a big size box that incorporated the source of electric energy, the electric motor and the mechanism of one way movement, something that made the system user non friendly.

At this period appeared the first efforts to introduce intramedullary distraction osteogenesis with the use of shape memory alloys as motive mechanisms.

The application of internal distraction osteogenesis using shape memory alloys has all the advantages of internal osteosynthesis. The only part of these mechanisms that is found externally is the activation mechanism that is connected by the necessary cables of activation with the interior of the bone where the internal distraction devised is placed.

The basic problem of all designs is the big constructional complexity of activation and control of shift of the two parts of the bone, something that makes this systems non user friendly and with continuous fractures and blockings of the elements of the mechanisms.

In the present invention that is described further down, all the advantages of the mechanism of internal distraction osteogenesis are combined with fundamental advantage, the simplicity of manufacture of the mechanism and the simplicity of operation via the restriction of moved elements.

### References

1. G. A. Ilizarov, Clin. Orthop. 250 (1990), 8.
2. M. A. Catagni Treatment of fractures nonunions and bone loss of the tibia with the Ilizarov method, Ed. A. Bi. Maiocchi 1998.
3. Operative principles of Ilizarov, Ed. A. Bi Maiocchi & J. Aronson 1991.
4. K. Otsuka, C.M. Wayman, Shape Memory Materials, Cambridge Un. Press, 1998.
5. Zadno et al. and In. Pelton et al. Proceedings 2nd International Conference on Shape memory and Superelastic Technologies (SMST); Pacific Grove, Mias 1997, 437-442.
6. A.M.M. Aalasma, E.E.G. Hekman, J. Stapert and H. Grootenboer, Design of an intramedullary leg legthening device with a shape memoty actuator, Technology and Health Care 7 (1999) 461-467.
7. V. Brailovski, F. Trochu Review of shape memory alloys medical applications in Russia, Biomed. Mater. Eng. 1996; 6 (4): 291-298.
8. K. Dai, Y. Chu Studies and applications of NiTi shape memory alloys in the medical field in China, Biomed. Mater. Eng. 1996; 6(4): 233-240.
9. S.A.Shabaloskaya On the nature of the biocompatibility and on the medical applications of NiTi shape memory and superelastic alloys. Biomed. Mater. Eng. 1996; 6(4): 267-289.
10. F. Trochu, V. Brailovski, M.A. Meunier, P. Terriault, Y.Y. Qian, Thermomechanical analysis of shape memory devices. Biomed. Meter. Eng. 1996; 6(6): 389-403.
11. J. Dean Cole, D. Justin, Tagus Kasparis, D. DeVlught, C. Knobloch, The intramedullary skeletal kinetic distractor (ISKD): first clinical results of a new intramedullary nail for lengthening of the femur and tibia, Injury, 2001; 32, (4),: 129-139.

### Revelation of invention

The invention of which the consolidation is requested, concerns an intramedullary nail of distraction osteogenesis that is constituted by two parts, each fixed to one from the two parts of the bone. The basic functional element of the system, which leads to the elongation of the parts of the bone, is based on the relative torsion of the two parts of the mechanism and it is constituted by shape memory alloys that are thermally activated.

Object of the invention is the use of thermal activated shape memory alloy as motive torsional mechanism for the relative turn and displacement of two parts of intramedullary nail of distraction osteogenesis.

Object of the invention is the use of a special screw (in left side is clockwise and in the right side is anti-clockwise) for the axial elongation of the two parts of the mechanism.

Object of the invention is the use of a special screw (in left side is clockwise and in the right side is anti-clockwise) to bear the compressive forces applied to the mechanism from the muscles and the body weight during walking.

Object of the invention is the use of a special screw (in left side is clockwise and in the right side is anti-clockwise) for the automatic control of the rotation of the mechanism to opposite time of the desirable which would lead to the reduction of the gap and minimization of the distance of the two parts of the bone.

Object of the invention is the use of a ratchet that deters to opposite shift of the desirable of the mechanism.

Object of the invention is the electronic system that allows the concretization of a circle of activation of the mechanism per 6 hours and lead to a total daily displacement of the two parts of the mechanism of 1mm that is given, separated in 4 equal steps of 0.25mm per 6 hours. This process of displacement has been fixed according to biomechanical studies that have preceded and led to most optimal result of elongation of the bone.

Object of the invention is the use of a spring that assists the shift of the parts of the mechanism for the restriction of required torsional susceptibility mainly at the first stages of loads based on the achieved partial zero loads because of the imposed force of the spring.

The mechanism is manufactured from stainless steel. The thermally activated shape memory alloy is a Nickel - Titanium alloy, which has been submitted in suitable surface wash for the displacement of Ni. The spring is manufactured from steel of springs.

The advantages of the proposed to consolidation intramedullary system of distraction osteogenesis are many if we compare them with previous corresponding solutions that have been proposed.

The torsional mechanism of displacement of the two parts of the system, as the whole manufacture is simpler, with less moving parts and consequently more economic and less functional problematic. The elements of fixation of the mechanism in the bone have simpler internal configuration and it can be manufactured in various sizes.

### List of Sketches

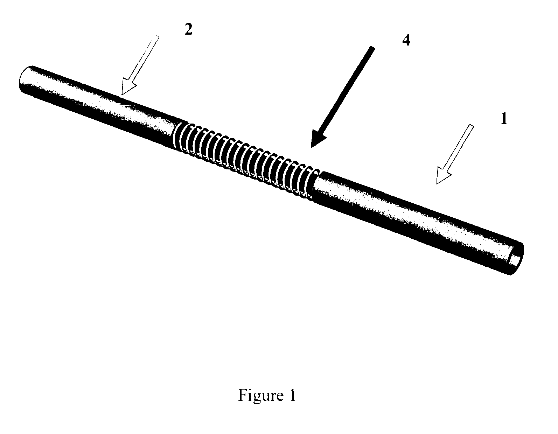

Figure 1: Schematic display of the intramedullary nail which use shape memory alloy to increase its length.

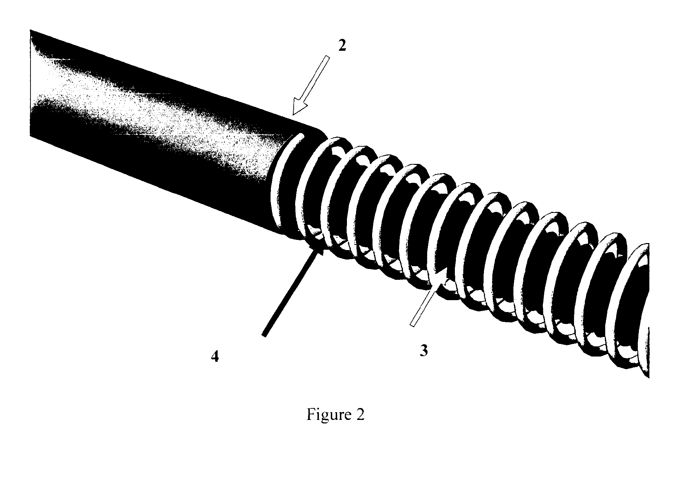

Figure 2: Schematic display of the screw and the spring that assists the elongation of the two parts of the nail

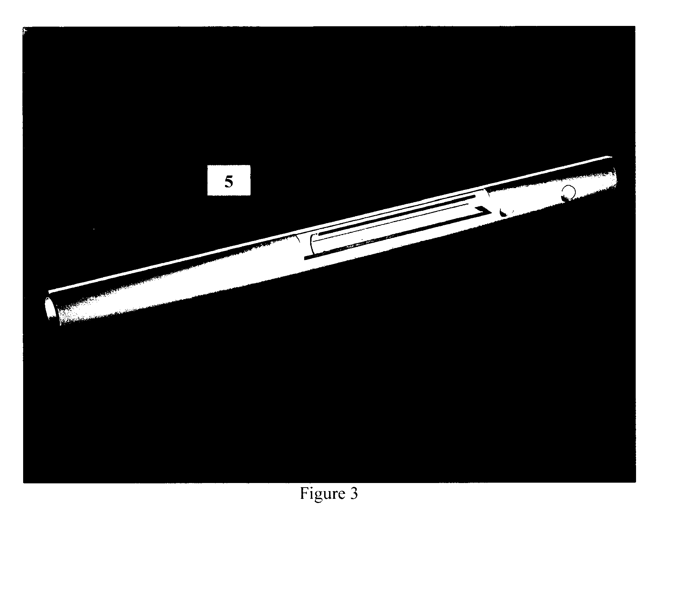

Figure 3: Schematic display of the shell in which is incorporated the mechanism of activation of the elongation.

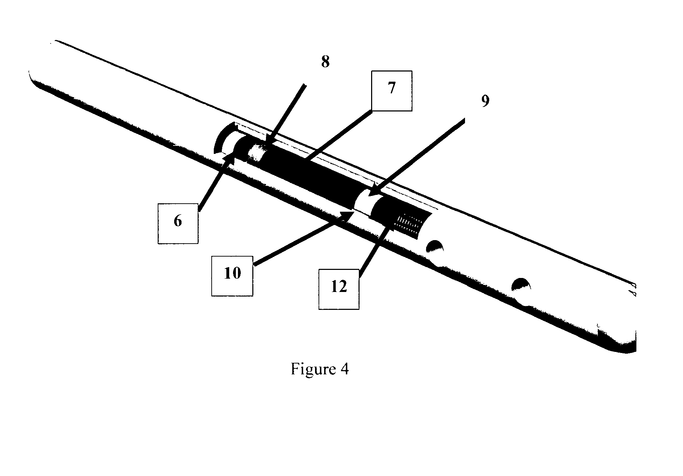

Figure 4: Schematic display of the mechanism which removes axially the two parts.

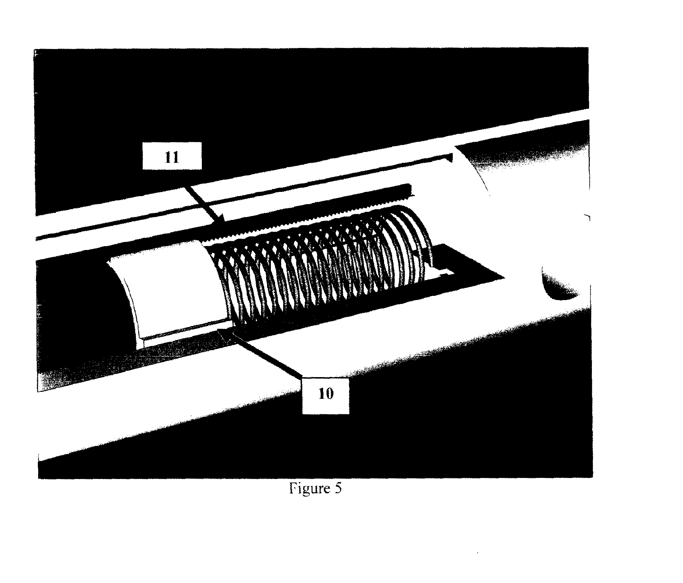

Figure 5: Detail of mechanism that imposes the continuous contact of the shape memory alloy and the ratchet.

### Description of invention

The presented invention is an intramedullary nail of variable length that can be used for the elongation of long bones either in cases of fractures with bone loss or diseases resulted in bone shortening. As appears in form 1, the intramedullary nail is constituted by two parts (1 and 2), (nutshells) that they aim to offer the essential axial and torsional stiffness to the mechanism based to the developing charges while walking. Simultaneously the two nutshells (1, 2) have the attribute to incorporate the mechanisms of activation and shift. The above mentioned parts bring incorporated mechanisms of activation of axial displacement of the two parts of the nail via torsion.

Parts 1 and 2 of the intramedullary nail are also fixed respectively to the two parts of the bone. Each part of the nail for functional reasons that will be explained later, bring interior and exterior engraved splines. The two parts of the nail are connected via a snail (3) which brings a clockwise screw to the one side and an anti-clockwise screw to the other. On the snail a spring (4) is incorporated which is compressed so, that it tends to remove the two parts of the nail and therefore limits the required torsional propensity that will remove these parts. The relative turn of the two parts of the nail is raised via a system of father key (5) that is placed inside a keyway and is engraved on the exterior surface of the nail, allowing with this way only axial shift of the two parts of the nail. Inside the two parts of the nail, two same but opposing turned mechanisms of torsional activation of axial displacement of these parts that one of them (the clockwise one) appears in form 2.

This mechanism consists of a cylindrical base (6) that is connected and immobilized at the end of the snail and on its frontal surface a rotary ratchet is attached, as shown in sketch 2. By thermally activated shape memory alloy of solenoid form (7) from its one immobilized inside a cylindrical base with configuration of a rotary ratchet (8) and collaborates with the ratchet (6) and since, the other end is immobilized inside a cylindrical base (9) that brings regional keyways where inside are placed wedges (10) that connect the part of the nail with the system of torsional activation. The cylindrical base (9) brings also a one way axial ratchet which collaborates with the corresponding ratchet (11) that is placed inside the suitably shaped keyways of the two parts of the nail and prevents the loss of contact of these parts (6) and (8) the rotary ratchet. Finally the device includes a spring (12) that pushes the mechanism inside the nail and ensures the continuous contact of the parts (6) and (8). The intramedullary nail is placed inside the bone, operating by inductively heated shape memory alloy.

### Description of operation

The activation of the inductive heating system increases the temperature of the shape memory alloy, and whenever this is heated at temperature above its activation temperature, it is turned to the direction such as to increase the length of the mechanism. When the rotation of the mechanism is completed, the inductive heating is interrupted and the shape memory alloy is frozen and, returns to its opposite/initial place. The rotary ratchet, during the cooling of the shape memory alloy, allows the return of the shape memory alloy to its initial place preventing simultaneously the rotation of the whole mechanism to the direction of reduction of the length. The spring which is located at the end of the nail pushes the entire mechanism to increase the length after the completion of its rotation. Simultaneously, the one way axial ratchet which is located at the end of the nail ensures continuous contact of the two parts of the ratchet. The movement of the screw that leads to the displacement of the two parts also leads to the increase of the length of the spring preventing, at the same time, the backwards motion of the rotary ratchet that compress the two pieces of the appliance. The above does not cause any problem to the operation of the mechanism because the role of the spring by removing the two parts of the intramedullary nail and by reducing the required force/of propensity of rotation at the first stage of application of intramedullary nail. As a result the forces that should be applied for the displacement of the two parts of the intramedullary nail and according to the bone, as well, are the largest.

## Claims

1. Claimed an intramedullary nail which has two parts (1, 2) each of which is fixed to the corresponding part of the long bone with screws. The two parts of the mechanism are axially deformed using a screw which in the one side is turned clockwise and on the opposite side anti-clockwise (3).By turning the screws the gap between the parts of the nail using the torsional power of the shape memory alloy can be increased (7).

2. Based on claim 1, the use of a special screw is claimed where on the one side is clockwise and on the opposite side is anti-clockwise (3) with suitably shaped base or incorporated base of one way ratchet (6, 8) for the axial deforming of the two parts of the intramedullary nail such as to overcome compressing forces applied to mechanism by the muscles. The use of the above mentioned screw allows controlling the elongation of the deformed parts of the nail.

3. Based on claim 1, a mechanism of torsional activation (7) is claimed, constituted of a metal base (8) fixed in one side of a thermal activated shape memory alloy which has been trained to give a torsional deform after its thermal activation and a metal ratchet in the opposite side of the shape memory alloy (9). The part of the metallic base cannot be turned inside of the nail because of a system which prevents all the other motion except the axial (11). The rotation of the thermal activated shape memory alloy using the ratchet leads to the rotation of the screw (3) that collaborates with the ratchet inside the nut - screw and leads finally to the abruption of the two parts of the nail.

4. Based on claim 1, a spring (4) is claimed, that helps the distraction of the two parts of the intramedullary nail which leads to limited required applied torsional forces from the mechanism that will lead in turn to the axial displacement of the parts of the nail.

5. Based on the claim 1, an induction heating system of the shape memory alloy and/or a system of direct heating via electric current is demanded, that leads to the activation of the mechanism of rotation every 6 hours. Therefore this results to a total daily distraction of the two parts of the intramedullary nail (also of the long bone which is fixed the intramedullary nail) of 1mm given in four steps of 0.25mm each.

6. Based on claim 1, the parts of the intramedullary nail (1, 2), the mechanism of movement and relative elements (6,8,9,10,11) are claimed, to be manufactured from stainless steel, or titanium alloy or biocompatible polymers or finally from a combination of these, while thermal activated shape memory alloy (7) that is "specially trained" in order to provide torsion. It can be constituted from titanium alloy and Nickel or from the previous alloys using another kind of materials. Finally the operational temperature of the above mentioned shape memory alloy is between 40 to 85° C.
